# EUROPEAN PATENT SPECIFICATION

(11) **EP 2 397 144 B1**
(45) Date of publication and mention of the grant of the patent: **31.12.2014**
(21) Application number: 11170127.2
(22) Date of filing: 16.06.2011
(51) Int. Cl.: A61K 31/7042, A61K 36/64, A61K 9/00, A61K 9/70, A61K 36/00, A61K 8/60, A61Q 19/00, A61P 25/28

(54) **Use of isoacteoside in preventing or treating amyloid beta peptide-associated diseases or conditions, and method for inhibiting formation, accumulation or aggregation of amyloid beta peptides**
Verwendung von Isoacteosid bei der Vorbeugung und Behandlung von Krankheiten und Störungen im Zusammenhang mit Amyloid-beta-Peptiden sowie Verfahren zur Hemmung der Bildung, Ansammlung und Anhäufung von Amyloid-beta-Peptiden
Utilisation d'un isoactéoside pour la prévention ou le traitement de maladies et troubles liés au peptide d'amyloïde bêta et procédé pour inhiber la formation, l'accumulation ou l'agrégation de peptides d'amyloïde bêta

(30) Priority: 16.06.2010 US 355169 P
(43) Date of publication of application: 21.12.2011
(73) Proprietor: Sinphar Tian-li Pharmaceutical Co., Ltd. (Hangzhou), Yuhang Economic Development Zone Hangzhou Zhejiang 311100 (CN)
(72) Inventor: Lin, Hang-Ching, Taipei City (TW); Tang, Jing-Jing, I Lan (TW); Su, Muh-Hwan, I Lan (TW); Huang, Young-Ming, I Lan (TW)
(74) Representative: Grünecker, Kinkeldey, Stockmair & Schwanhäusser

(56) References cited:
- WO-A1-2004/014410
- US-A1- 2004 162 246
- DATABASE BIOSIS [Online] BIOSCIENCES INFORMATION SERVICE, PHILADELPHIA, PA, US; May 2006 (2006-05), LIU FENG-XIA ET AL: "The effects of glycosides of cistanche on learning and memory in beta-amyloid peptide induced Alzheimers disease in mice and its possible mechanism", XP002654644, Database accession no. PREV200600523442 & ZHONGGUO YAOLIXUE TONGBAO, vol. 22, no. 5, May 2006 (2006-05), pages 595-599, ISSN: 1001-1978
- DATABASE WPI Week 200961 Thomson Scientific, London, GB; AN 2009-N27690 XP002654645, & JP 2009 196905 A (EISHIN SHOJI KK) 3 September 2009 (2009-09-03)
- DATABASE WPI Week 200769 Thomson Scientific, London, GB; AN 2007-731327 XP002654646, & JP 2007 191416 A (EISHIN SHOJI KK) 2 August 2007 (2007-08-02)
- DATABASE EPODOC [Online] EUROPEAN PATENT OFFICE, THE HAGUE, NL; 21 July 2004 (2004-07-21), XP002654647, & KR 2004 0064830 A (ELCOM SCIENCE CO LTD) 21 July 2004 (2004-07-21)
- LI ET AL: "Pedicularioside A from Buddleia lindleyana inhibits cell death induced by 1-methyl-4-phenylpyridinium ions (MPP<+>) in primary cultures of rat mesencephalic neurons", EUROPEAN JOURNAL OF PHARMACOLOGY, ELSEVIER SCIENCE, NL, vol. 579, no. 1-3, 30 October 2007 (2007-10-30), pages 134-140, XP022410154, ISSN: 0014-2999, DOI: DOI:10.1016/J.EJPHAR.2007.10.052
- WANG H ET AL: "Acteoside protects human neuroblastoma SH-SY5Y cells against beta-amyloid-induced cell injury", BRAIN RESEARCH, ELSEVIER, AMSTERDAM, NL, vol. 1283, 4 August 2009 (2009-08-04), pages 139-147, XP026350245, ISSN: 0006-8993 [retrieved on 2009-06-09]
- JIANG Y ET AL: "Analysis of chemical constituents in Cistanche species", JOURNAL OF CHROMATOGRAPHY, ELSEVIER SCIENCE PUBLISHERS B.V, NL, vol. 1216, no. 11, 13 March 2009 (2009-03-13), pages 1970-1979, XP025992515, ISSN: 0021-9673, DOI: DOI:10.1016/J.CHROMA.2008.07.031 [retrieved on 2008-07-18]
- DATABASE EPODOC [Online] EUROPEAN PATENT OFFICE, THE HAGUE, NL; 8 November 2006 (2006-11-08), XP002654745, & CN 1 857 519 A (CHINA OCEANOLOGY UNIV [CN]) 8 November 2006 (2006-11-08)
- DATABASE WPI Week 201048 Thomson Scientific, London, GB; AN 2010-F56787 XP002654746, & CN 101 703 125 A (XINJIANG ECOLOGY & GEOGRAPHY INST CHINES) 12 May 2010 (2010-05-12)
- DATABASE WPI Week 200703 Thomson Scientific, London, GB; AN 2007-021459 XP002654747, & JP 2006 312594 A (TAISHO PHARM CO LTD) 16 November 2006 (2006-11-16)
- DATABASE EMBASE [Online] ELSEVIER SCIENCE PUBLISHERS, AMSTERDAM, NL; 7 May 2005 (2005-05-07), LI X -L ET AL: "Effects of fufang jinsiwei versus placebo, saline and Aricept on the expression of protein 95 in post-synapse dense area of brain tissue in rats with Alzheimer disease", XP002654748, Database accession no. EMB-2005502417 & CHINESE JOURNAL OF CLINICAL REHABILITATION 20050507 CN, vol. 9, no. 17, 7 May 2005 (2005-05-07), pages 139-141, ISSN: 1671-5926
- ADAMS ET AL: "Plants traditionally used in age related brain disorders-A survey of ethnobotanical literature", JOURNAL OF ETHNOPHARMACOLOGY, ELSEVIER SCIENTIFIC PUBLISHERS LTD, IE, vol. 113, no. 3, 25 September 2007 (2007-09-25), pages 363-381, XP022230985, ISSN: 0378-8741, DOI: DOI:10.1016/J.JEP.2007.07.016

## Description

### CROSS-REFERENCE TO RELATED APPLICATIONS

This application claims priority benefit from US Provisional Application No. 61/355,169, filed June 16, 2010.

### BACKGROUND OF THE INVENTION

### 1. Field of the Invention

The present invention generally relates to the use of isoacteoside or a pharmaceutically acceptable salt thereof in preventing or treating amyloid β peptide (amyloid β peptide; Aβ) associated diseases or conditions.

### 2. Description of the Prior Art

Alzheimer's disease is considered to be related to accumulation of peptides of 39-43 amino acids, and such peptides are called as amyloid β peptides, which are hydrolysis products of amyloid precursor protein (APP). Among the Aβ, Aβ1-40 is the most abundant form, while Aβ1-42 is more toxic to neurons and highly fibrillogenic and is considered as the most relevant Aβ form to Alzheimer's disease. Aβ monomers can form soluble Aβ oligomers through oligomerization, and further form insoluble fibrils or senile plaques extracellularly.

It is generally considered that Aβ-associated diseases or conditions comprise Down syndrome, hereditary cerebral hemorrhage with amyloid (HCHWA) Dutch, Parkinsonism-dementia complex on Guam (Clinical Neurology and Neurosurgery (1990) 92: 305-310); Cerebral amyloid angiopathy (J. Neuropath. Exp. Neuro. (2002) 61: 282-293); inclusion body myositis (Neurology (2006) 66: 65-68); frontotemporal dementia (Neuroreport (2002) 13-5: 719-723); age-related macular degeneration (Experimental Eye Research (2004) 78: 243-256); Pick's disease (Neuroscience Letters (1994) 171: 63-66), and others.

Aβ-associated diseases or conditions as described are generally related to formation, accumulation or aggregation of Aβ, leading to abnormal quantity of Aβ or Aβ aggregates present in organisms caused by congenital factors (e.g., inheritance) or acquired factors (e.g., aging or environmental effects).

It is generally believed that inhibition of Aβ formation, accumulation or aggregation can be used as an approach for effectively preventing or treating Alzheimer's disease or other Aβ-associated diseases or conditions.

### SUMMARY OF THE INVENTION

It is the object of the present invention to provide an active ingredient for use in preventing or treating a disease or condition associated with amyloid β peptides in an individual, wherein said disease or condition is mild cognitive impairment, Lewy body dementia, Down syndrome, Hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, or Pick's disease. Such active ingredient can be used as an additive in food, drinks, chewing gums, patches or skin care products.

Said object is achieved by isoacteoside or a pharmaceutically acceptable salt thereof for use according to appended claim 1.

Preferred embodiments of the present invention are set forth in the dependent claims.

To better understand the above and other objects, features and advantages of the present invention, the present invention will be described in detail below taken from the examples with reference to the annexed drawings.

### BRIEF DESCRIPTION OF THE DRAWINGS

Fig. 1 shows the percentage level of extracellular Aβ1-40 of each well, the results indicating the test sample D (isoacteoside) possesses significant activity on reducing extracellular Aβ1-40 accumulation;
Fig. 2 shows the percentage level of intracellular Aβ1-40 of each well;
Fig. 3 shows the effects of the test samples on APP expression;
Fig. 4 shows the effects of the test samples on Aβ1-40 degradation
Fig. 5 shows the effects of the test samples on Aβ1-40 oligomerization;
Fig. 6 illustrates the processes of formation, clearance and aggregation of Aβ;
Fig. 7 illustrates the experimental schedule for animal studies;
Fig. 8 shows the effects of the test samples on the exploration behavior performance of the rats;
Fig. 9 shows the effects of the test samples on the passive avoidance response performance of the rats;
Fig. 10 shows the effects of the test samples on the water maze spatial performance of the rats;
Fig. 11 shows the effects of the test samples on the water maze probe test performance of the rats;
Fig. 12A-E shows the immunohistochemical staining results of the rats;
Fig. 13A shows the levels of acetylcholine in the brain cortex and hippocampus of the rats;
Fig. 13B shows the levels of choline in the brain cortex and hippocampus of the rats;
Fig. 14A shows the activities of acetylcholinesterase in the brain cortex of the rats; and
Fig. 14B shows the activities of acetylcholinesterase in the brain hippocampus of the rats.

### DETAILED DESCRIPTION

Various diseases caused by Aβ have a common feature: formation of Aβ aggregates. These Aβ aggregates present in shapes such as fibrils or plaques, and deposit in systems, organs, tissues or body fluids of organisms, causing various diseases or conditions. It is therefore supposed that inhibition of Aβ formation, accumulation or aggregation can be used as an approach for effectively preventing or treating Aβ-associated diseases or conditions.

In view of the above, the present specification discloses using isoacteoside having a structure shown below or a pharmaceutically acceptable salt thereof as an active ingredient for inhibiting (e.g., reducing or preventing) Aβ formation, accumulation or aggregation, and in particular Aβ extracellular formation, accumulation or aggregation.

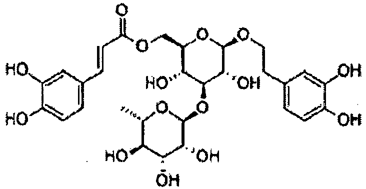

U.S. patent 7,087,252 B2 discloses a medicinal preparation, which comprises 10-70 weight percent of echinacoside and 1-40 weight percent of acteoside, prepared from fleshy stems of *Cistanche tubulosa* (Schenk) Wight, and is provided against senile dementia. Isoacteoside and various other phenylethanoid glycosides are known to be included in the medicinal preparation.

Liu Feng-Xia et al. ("The effects of glycosides of cistanche on learning and memory in beta-amyloid peptide induced Alzheimers disease in mice and its mechanism", Biosciences Information Service, Philadelphia, PA, US; May 2006) describe the protective effects of glycosides of cistance on an animal model of Alzheimer's disease.

In the present invention, hydrates or other solvates of isoacteoside are deemed as functional equivalents of isoacteoside.

When a pharmaceutically acceptable salt of isoacteoside is administered to an individual, the pharmaceutically acceptable salt generally provides equivalent or similar therapeutic effects as isoacteoside, and is physiologically tolerable without causing adverse side effects such as allergy. The pharmaceutically acceptable salt of isoacteoside may comprise but is not limited to iron, calcium, and magnesium salts.

The term "prevent" used herein means avoiding or delaying occurrence of a disease or condition in organisms. The term "treat" used herein means slowing or stopping progress of a disease or condition, or making an individual return back to his improved or normal status.

The term "amyloid β peptide (Aβ)-associated diseases or conditions" generally refers to those diseases or conditions that occur relating to formation, accumulation or aggregation of Aβ, and particularly refers to the diseases or conditions that are caused by Aβ. When abnormal formation, accumulation or aggregation is found in a certain proportion of individuals with certain diseases or conditions, the diseases or conditions can be considered as being associated with Aβ. In addition, when Aβ aggregates somewhere that is close to occurrence of pathological features affected in certain diseases or conditions, the diseases or conditions can be also considered as being associated with Aβ.

The active ingredient for Aβ inhibition can be used as an additive in food or drinks to facilitate the use of inhibiting formation, accumulation or aggregation of Aβ.

The drugs or pharmaceutical compositions for use in treating Aβ-associated diseases or conditions, which comprise isoacteoside or the pharmaceutically acceptable salt thereof as an active ingredient, can comprise suitable carriers, diluents or excipients, and can be present in powders, granules, tablets, troches, pills, capsules, solutions, suspensions, creams, ointments, gels, aerosols, suppositories, patches or any other desired forms. The drugs or pharmaceutical combinations described above can be administered via oral, topical, parenteral, dermal, intranasal, ocular, intraocular or other routes.

In the context, the term "or" is generally defined as "and/or", unless it is otherwise specified.

Along with the aging of the population, dementia, which is related to aging, has become one of the major concerns in the present medical researches related to senile diseases. Alzheimer's disease is the most common type of dementia. Alzheimer's disease is a chronic progressive neurodegenerative disease, characterized in that patients gradually lose their cognition ability and show abnormal behavior, followed by a possible loss of verbal and motion abilities, and thus Alzheimer's disease easily makes a great impact on the patient's and his/her family's life qualities. In view of the trend of the number of patients suffering Alzheimer's disease gradually increasing, causing severe burdens on the patient's family and the society, the present invention selects the amyloid beta peptides (Aβ) which are more related to Alzheimer's disease for carrying out experiments.

**Table 1: Test samples**

| Symbol | Test sample | Purity (%) | Source |
|---|---|---|---|
| A | Preparation containing phenylethanoid glycosides | - | available from Tianlife® (referring to the preparation disclosed in US 7087252B2) |
| B | Echinacoside | 99 | Sinphar Lab. |
| C | Acteoside | 97 | Sinphar Lab. |
| D | Isoacteoside | 97 | Sinphar Lab. |

The chemical structures of echinacoside, acteoside and isoacteoside are shown below:
Echinacoside: 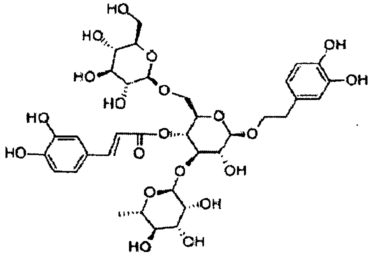
Acteoside: 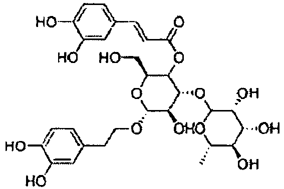
Isoacteoside:

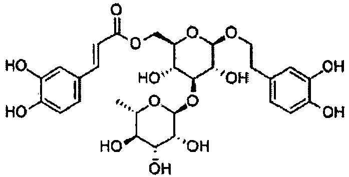

### Example 1: Neuroblastoma cell culture

Wild-type human neuroblastoma cells (SH-SY5Y) were cultured in Eagle's Minimum essential Medium (EMEM) / Ham's F12 medium (1:1 mixture) (containing 10% FBS, 10 units/ml penicillin, 10µg/ml Streptomycin). Wild-type mouse neuroblastoma Neuro-2a cells were cultured in minimum essential medium (MEM) (containing 10% FBS, 10 units/ml penicillin, 10µg/ml Streptomycin).

### Example 2: The effect of each test sample on extracellular Aβ1-40 accumulation

The medium of the wild-type human neuroblastoma SH-SY5Y cells in Example 1 were switched into chemical defined medium (EMEM/F12 medium (Cat.No.12500-062), Hepes 5mM, Glucose 0.6%, NaHCO₃ 3mM, Glutamine 2.5 mM, Insulin 25 µg/ml, Transferin 100 µg/ml, Progestrone 20 nM, Putrescine 60 µM, Sodium selenite 30 nM, Heparin 2 µg/ml). Each well contained 1x10⁵ SH-SY5Y cells in 300 µl of culture medium. Thirty minutes later, each well was treated with the test samples A-D given in Table 1 respectively at a concentration of 50 µg/ml for 24 hours. After that, the level of Aβ1-40 in the medium of each well was analyzed by Human Aβ1-40 immunoassay kits (Catalog # KHB3482 Invitrogen).

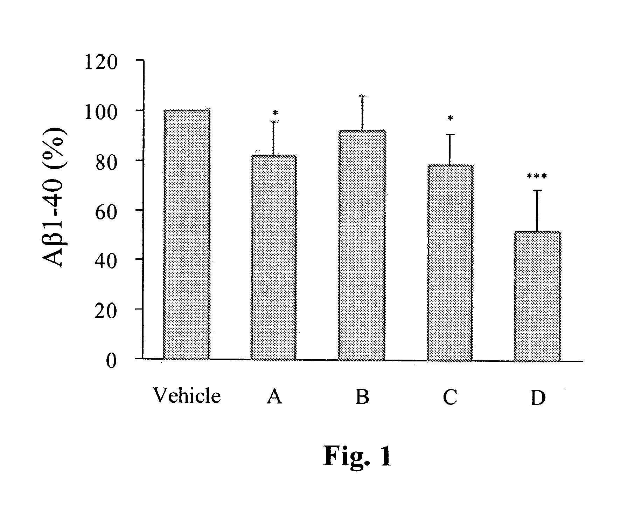

Human neuroblastoma SH-SY5Y cells cause extracellular accumulation of Aβ. Fig. 1 shows the percent level of Aβ1-40 in the medium of each SH-SY5Y well, based on the percentage level in Vehicle control group which is not treated with any test sample. The results were shown in mean ± standard deviation (SD) form. Significant difference between the Vehicle control group and the test sample-treated groups were indicated by *., P<0.05, *.*, P<0.01, and. ***., P<0.001.

Referring to Fig. 1, in comparison with the Vehicle control group, the test samples A (preparation containing phenylethanoid glycosides) and C (acteoside) reduced the level of Aβ1-40 by about 20%, and D (isoacteoside) reduced the level of Aβ1-40 by about 47.44 ± 16.62%. The results in Fig. 1 suggest that the test sample D (isoacteoside) possesses significant activity on reducing extracellular Aβ1-40 accumulation.

### Example 3: The effect of each test sample on intracellular Aβ1-40 accumulation

Each well contained 1x10⁵ SH-SY5Y cells in 300 µl of culture medium. After the SH-SY5Y cells of each well were individually treated with the test samples A-D at a concentration of 50 µg/ml, cell homogenate was individually prepared from cells of each well, and the amount of Aβ1-40 in the medium of each well was determined with Human Aβ1-40 immunoassay kits (Catalog # KHB3482 Invitrogen).

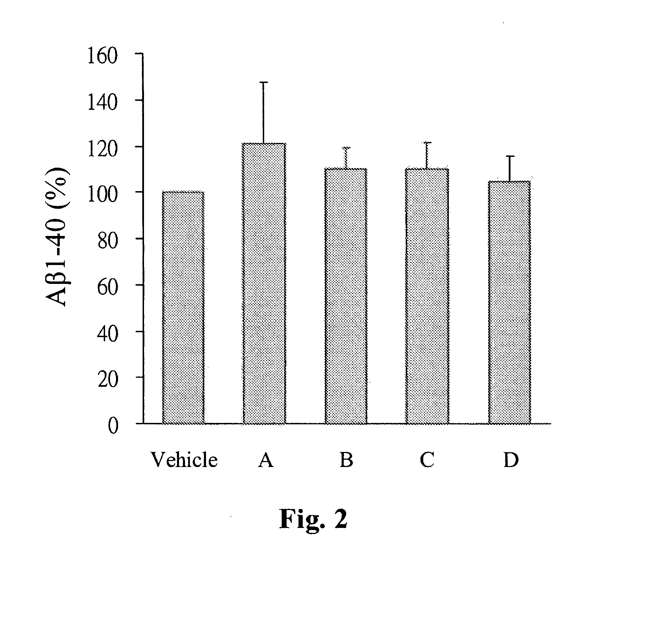

Fig. 2 shows the percentage level of intracellular Aβ1-40 of SH-SY5Y well, based on the Vehicle control group which is not treated with any test sample. Referring to Fig. 2, it is indicated that the test samples A-D do not significantly cause intracellular accumulation of Aβ.

### Example 4: The effect of each test sample on APP expression

Each well contained 1x10⁵ SH-SY5Y cells in 300 µl of culture medium. After the SH-SY5Y cells of each well were individually treated with the test samples A-D at a concentration of 50 µg/ml for 24 hours, the cells were homogenized in homogenize buffer (50 mM Hepes pH 7.5, 1 mM EDTA, 150 mM NaCl, 1% NP-40, 1 mM PMSF, 5 µg/ml aprotinin, 10 µg/ml leupeptin). Cell debris was removed by centrifugation. Proteins were separated by SDS-polyacrylamide gel electrophoresis and were then transferred to PVDF membrane, followed by evaluating holoAPP amounts by immunoblot (antiAβ1-17, 6E10 or anti-APP C-terminal antibody)

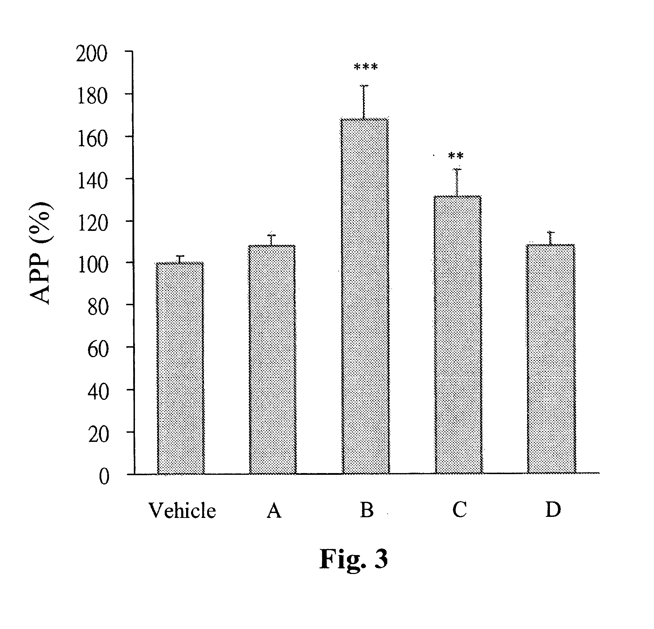

Fig. 3 shows the effects of the test samples A-D on APP expression in the cells, expressing as percentage, based on the Vehicle control group which is not treated with any test sample. As shown in Fig. 3, it is seen that the test samples A-D do not down regulate APP expression.

### Example 5: The effect of each test sample on extracellular Aβ1-40 degradation

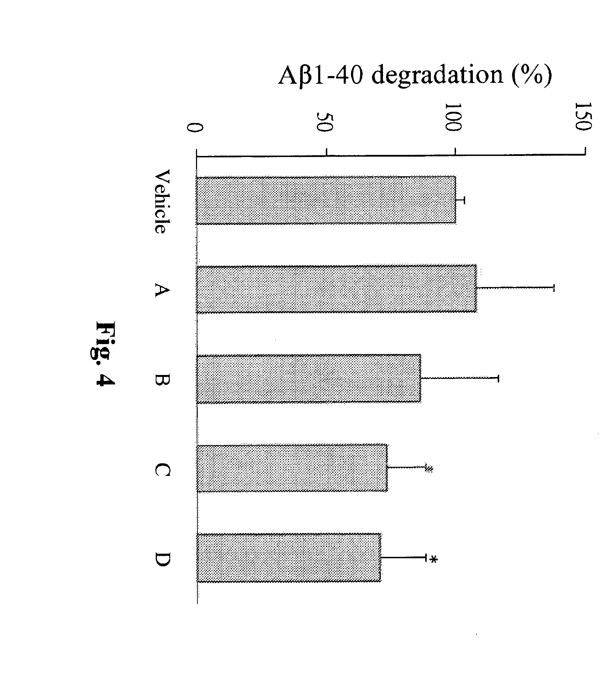

Mouse neuroblastoma Neuro-2a cells will release Aβ-degrading enzymes in the chemical defined medium, but will not produce detectable amount of Aβ in the medium. The Neuro 2a cells were incubated in the medium for 24 hours, and then the medium were drawn out without cells. The medium were individually treated with the test samples A-D at a concentration of 50 µg/ml and 10 ng synthetic Aβ1-40 for 24 hours, and then the effects of each test sample on promoting the enzymes in the medium were examined. The remaining Aβ1-40 amount of each well was analyzed by Human Aβ1-40 Immunoassay kits (Catalog # KHB3482 Invitrogen), expressing as percentage, as compared with the Vehicle control group. Referring to Fig. 4, the test sample D (isoacteoside) does not accelerate Aβ degradation.

### Example 6: The effect of each test samples on Aβ1-42 oligomerization

Dried Human Aβ1-42 was taken out from the refrigerator and equilibrated to room temperature. Aβ1-42 was dissolved in 1,1,1,3,3,3-Hexa-fluro-2-propanol (HFIP) to a concentration of 1mM, and was then placed at room temperature for one hour. The Aβ1-42/HFIP solution was aliquoted by Hamilton syringe, and was then dried under a stream of nitrogen gas, followed by storing at a temperature of -20°C. Aβ1-42 treated with HFIP was dissolved in PBS, and was vibration-incubated with treatment of each test sample at a concentration of 50 µg/ml and at 4°C for 24 hours to prepare Aβ1-42 oligomers. The level of Aβ1-42 oligomerization was analyzed by thioflavin T fluorescence (Ex=450 nm, Em=482 nm).

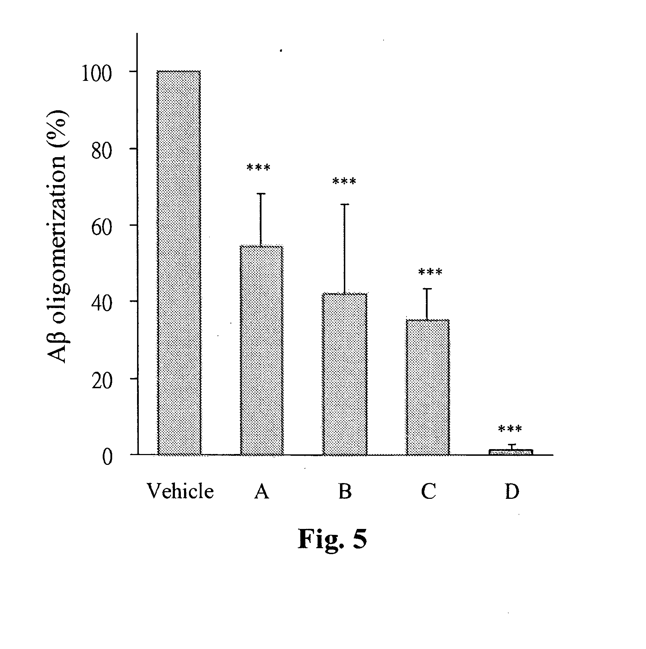

Fig. 5 shows the effects of the test samples A-D on Aβ1-40 oligomerization in percentage. Referring to Fig. 5, the test samples A (preparation containing phenylethanoid glycosides), B (echinacoside), C (acteoside) and D (isoacteoside) were found to possess activities on inhibiting Aβ1-42 oligomerization, wherein isoacteoside (D) was able to significantly inhibit Aβ1-42 oligomerization by 98.93 ± 1.70%. That is to say, isoacteoside (D) can significantly inhibit Aβ1-42 oligomerization, and further inhibit Aβ from forming fibrils or senile plaques.

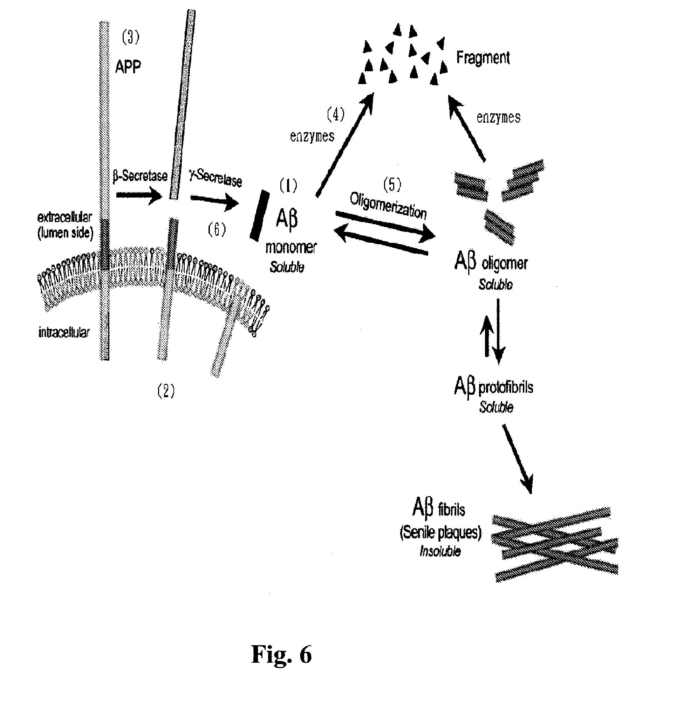

Fig. 6 illustrates the processes of formation, clearance and aggregation of Aβ (Pharmacology & Therapeutics (2005) 108: 131). It is shown that the extracellular Aβ accumulation (1) in the medium will be affected by intracellular Aβ accumulation (2), APP expression (3), Aβ degradation (4), Aβ oligomerization (5) and Aβ formation (6). According to the results from cell studies in Examples 2 to 6, as compared to the test samples A-C, isoacteoside (D) is effective in reducing the extracellular Aβ accumulation (1) in the medium and is able to significantly inhibit Aβ oligomerization (5), which is supposed to inhibit Aβ aggregation in advance. Besides, the effect of isoacteoside (D) on reducing extracellular Aβ accumulation (1) is not due to isoacteoside enhancing the intracellular Aβ accumulation (2), reducing the APP expression (3) of the cells, or enhancing extracellular Aβ degradation (4). It is therefore suggested that the effect of isoacteoside (D) is directly on the reducing of Aβ formation.

In summary, isoacteoside (D) or its pharmaceutically acceptable salts can be used as an active ingredient for inhibiting formation, accumulation or aggregation of Aβ. Therefore, it is expected that isoacteoside (D) can be provided for effectively inhibiting neuronal damage or apoptosis caused by Aβ, and further for retaining, improving or restoring learning or memory abilities. In addition, according to those results above, isoacteoside (D) or the pharmaceutically acceptable salts thereof can be provided for preventing or treating Aβ3-associated diseases or conditions, as well as for inhibiting formation, accumulation or aggregation of Aβ.

The described Aβ-associated diseases or conditions are mild cognitive impairment, Lewy body dementia, Down syndrome, hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration and Pick's disease. In addition, even though the described Aβ is exemplified by Aβ1-40 at most or highly fibrillogenic Aβ1-42, the Aβ can also comprise other peptide fragments.

Among the test samples above, isoacteoside (D) possesses excellent effects on reducing the formation, accumulation or aggregation of Aβ. In the following Examples 7 to 11 illustrated below, male Sprague-Dawley (SD) rats, weighed 250-300 gm, were obtained from BioLASCO Taiwan Co. Ltd.. The SD rats were infused intracerebroventricularly with Aβ1-42 to cause neuronal damage, affecting their memory and learning ability, and leading to form senile plaque-like aggregates in the rat's brain, being used as an animal model of Alzheimer's disease. Alzheimer's disease animal model induced by Aβ can be referred to, for example, Nabeshima et al. (Neuroscience Letters (1994) 170: 63-66) (British Journal of Pharmacology (1999) 126: 235-244).

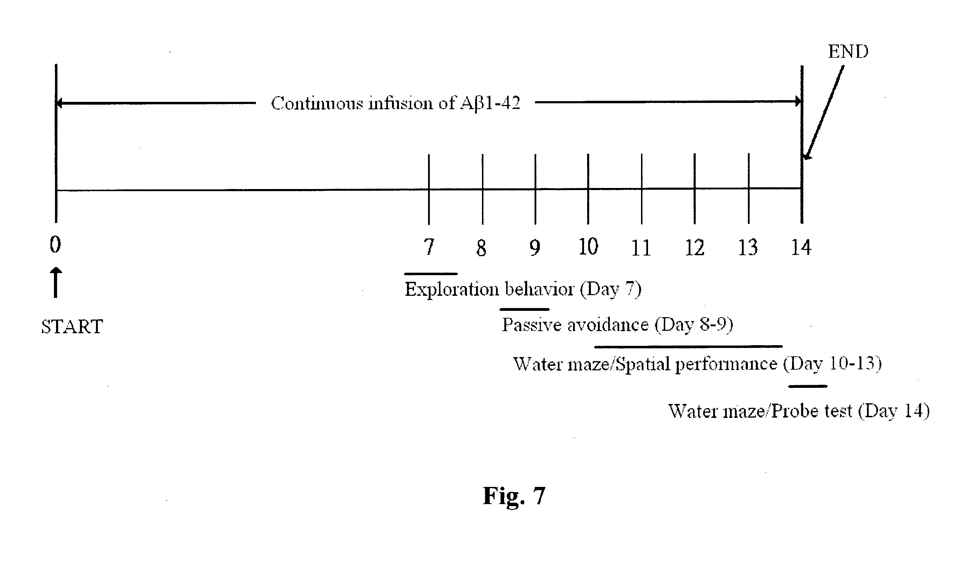

The rats were anesthetized for implantation of an infusion cannula into the left cerebral ventricle thereof. After sewing up the incisions, the rats were returned to their cages. Fig. 7 illustrates the experimental schedule for animal studies. The exploration behavior tests were carried out on day 7, the passive avoidance learning tests were carried out on day 8 and 9, the water maze tests were carried on day 10 to 13, and the probe tests were carried out on day 14, after the start of Aβ1-42 infusion. The experimental groups were shown in Table 2. The Sham group was treated with TFA solution (64.9% sterile ddH2O, 35% acetonitrile, 0.1 % trifluoroacetic acid), and the other experimental groups were treated with 0.5 µl of Aβ1-42 dissolved in TFA solution per hour, corresponding to 300 pmol/12µl of Aβ1-42 (Tocris 1428 ; MW : 4514.08) per day. The test samples were administered orally to the rats, 1 hour before the tests (i.e., the exploration behavior tests, the passive avoidance learning tests, and the water maze tests) throughout the experimental period. Table 2 shows the conditions for each experimental group, wherein D is isoacteoside shown in Table 1, and Aricept is a commercial drug for treating dementia such as Alzheimer's disease. All the test samples were prepared freshly with deionized distilled water (ddH2O) every day, and were administered orally by stomach tube.

In Examples 7 to 11, the results were shown in mean ± standard deviation (SD) form. Significant difference between the Aβ1-42 control group and the other experimental groups were indicated by *., P<0.05, *.*, P<0.01, and. ***., P<0.001.

All rats were sacrificed and their brains were removed, cut into slices and stained. Neurotransmitters were also tested.

| Groups | Inducer (intracerebroventricular infusion) | Test sample (administered orally by stomach tube) | Rats |
|---|---|---|---|
| Sham group | TFA solution | ddH2O | 12 |
| Aβ1-42 control group | Aβ1-42 | | 12 |
| D 2.5 mg/kg | | D 2.5 mg/kg | 12 |
| D 5 mg/kg | | D 5 mg/kg | 12 |
| Aricept | | Aricept 0.75 mg/kg | 12 |

### Example 7: Exploration behavior tests and apparatus

An exploration behavior test apparatus consisted of a box (40cm x 40cm x 40cm) and a stainless steel bottom board with 16 holes of a diameter of 3cm, wherein these holes were spaced 4 cm apart and were at a distance of 7 cm from the side edge. The entry counts of each rat were recorded for 10 minutes.

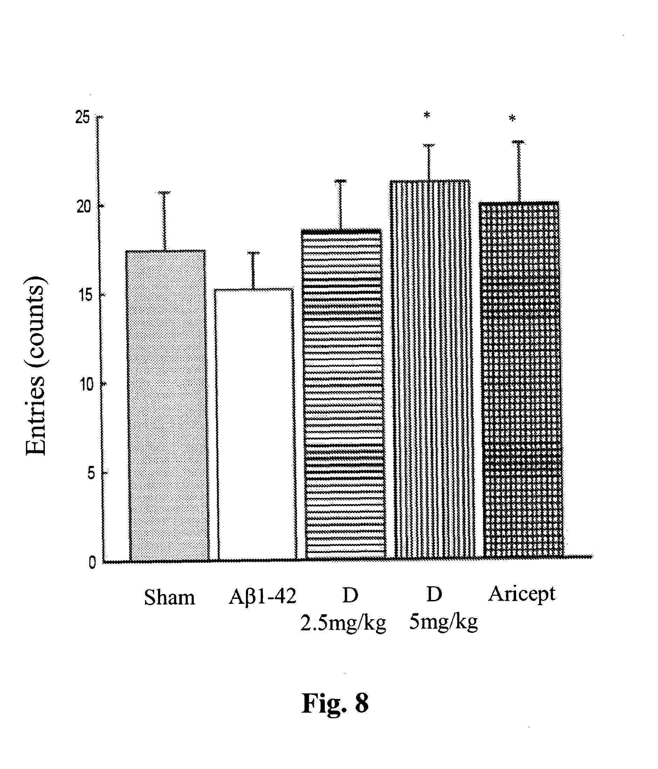

Fig. 8 shows results of the exploration behavior tests performed by the rats according to Table 2. Referring to Fig. 8, intracerebroventricular infusion of Aβ1-42 decreases the exploratory activities of the rats, and the test sample D (isoacteoside) and Aricept are effective in improving the exploratory activities of the rats, wherein the test sample D (isoacteoside) 5mg/kg shows better effects than Aricept 0.75mg/kg.

### Example 8: Passive avoidance response tests

A passive avoidance apparatus is consisted of a light compartment, a dark compartment and a guillotine door between the two compartments.

Each rat was placed in the light compartment while the guillotine door was open. The rats, which entered into the dark compartment within 90 seconds, were selected to be tested in this experiment.

On the training trial, each of the selected rats was individually put in the light compartment while the guillotine door was open. Upon entering the dark compartment, the guillotine door was closed, and an electrical shock was given to the rat in the dark compartment through the floor for five seconds. After that, the rat was removed from the dark compartment and was put back into its home cage.

Twenty four hours after the training trial, the rats were individually put in the light compartment while the guillotine door was open, and the step-through latency (STL) of each rat was recorded.

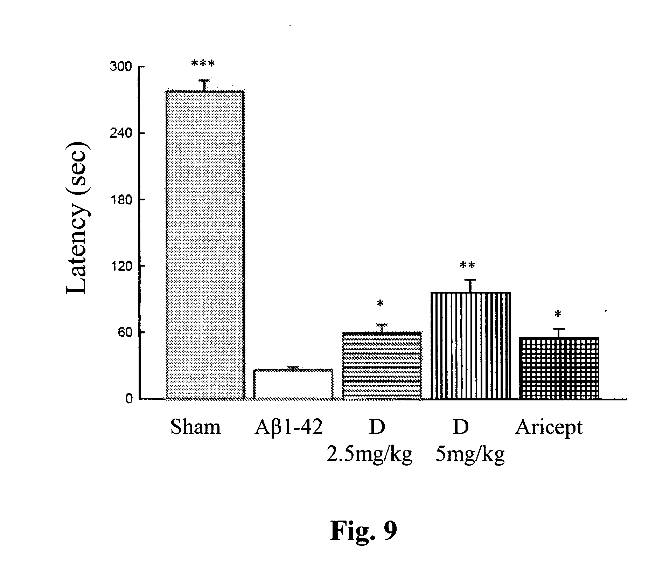

Fig. 9 shows the passive avoidance response results of the rats according to Table 2. Referring to Fig. 9, intracerebroventricular infusion of Aβ1-42 causes a significant impairment of the passive avoidance learning response for the rats, and the test sample D (isoacteoside) and Aricept reverse the passive avoidance learning impairment of the rats caused by intracerebroventricular infusion of Aβ1-42. As shown in Fig. 9, the test sample D (isoacteoside) 2.5 mg/kg shows equivalent effect with Aricept 0.75mg/kg, and the test sample D (isoacteoside) 5 mg/kg shows better effects than Aricept 0.75mg/kg.

### Example 9: Water maze tests

In this example, the water maze pool was divided into four quadrants, and a hidden platform was located in the fourth quadrant and was submerged 1.0 cm below the surface of water. Each rat was given two trials with an interval of four hours per day, two minutes for each trial to find the hidden platform. The first trial was performed for the spatial performance test. Each rat was allowed to swim a maximum of 120 seconds to find the hidden platform. When successful, the rat was allowed a 30-second rest period on the platform. If unsuccessful within the aborted time period, the rat was given a score of 120 seconds and then put on the platform, allowing a 30-second rest period. The spatial performance tests were performed for four consecutive days. On the fifth day, the hidden platform was taken away from the water maze pool, and each rat was placed in the first quadrant and was given a swimming period of 60 seconds. The time for each rat spent in the quadrant where the hidden platform located was recorded during the probe test.

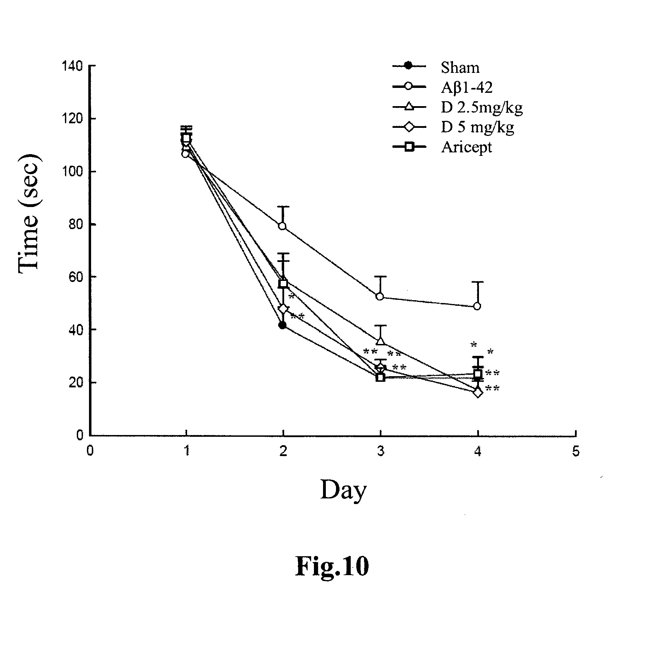

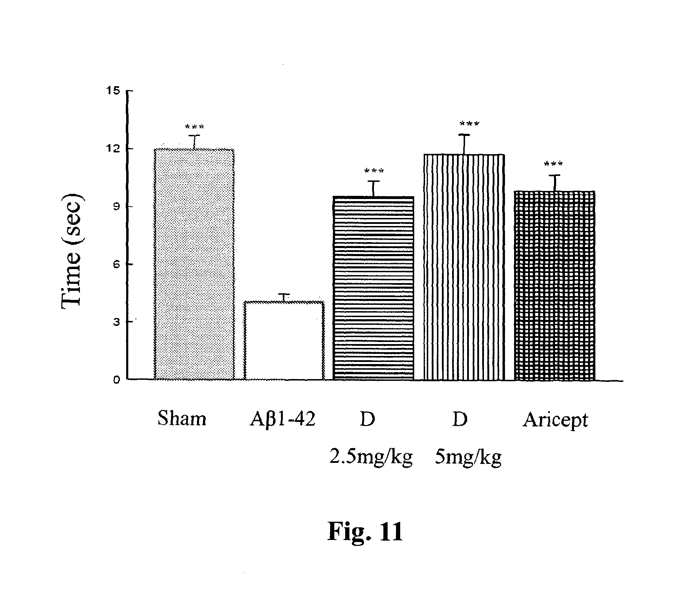

Fig. 10 shows results of the spatial performance tests performed by the animals in Table 2, and Fig. 11 shows results of the probe tests performed by the animals in Table 2. Referring to Fig. 10 and Fig. 11, it is shown that deficits of the water maze spatial performance and the probe test caused by Aβ1-42 can be improved by the test sample D (isoacteoside) and Aricept, and the test sample D (isoacteoside) 5 mg/kg shows better effects than Aricept 0.75mg/kg.

### Example 10: Immunohistochemical staining of rat's brain

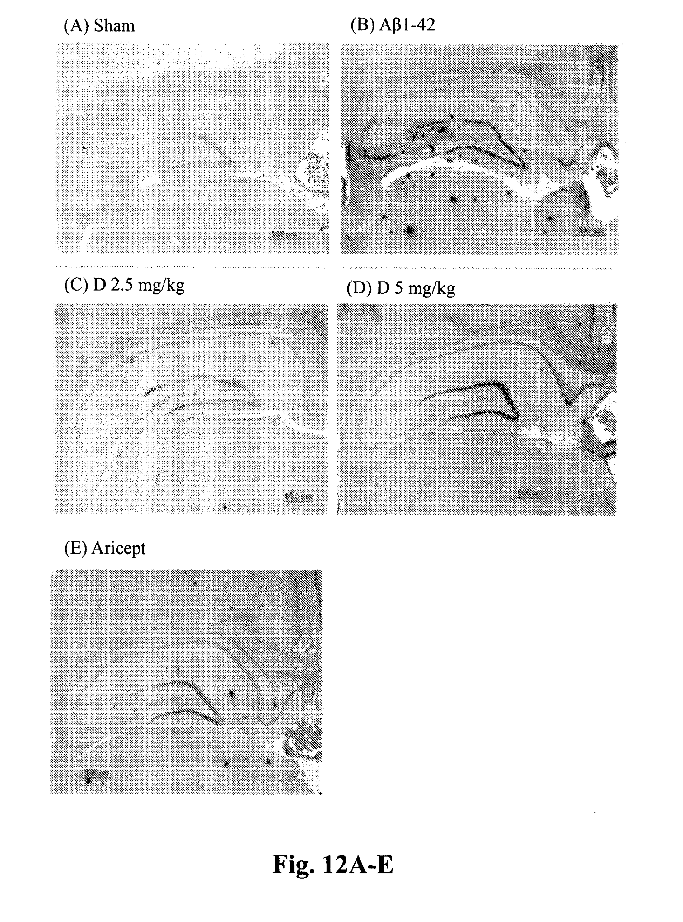

After the water maze performance tests, all rats were decapitated and their brains were rapidly removed from the skull, and then immunohistochemical staining for Aβ1-42 in the hippocampus section of their brains. Fig. 12A-E shows immunohistochemical staining results. In Fig. 12B, a number of plaques were found in the brain of the rat continuously infused with Aβ1-42. In Fig. 12C and Fig. 12D, few plaques were found in the brain of the rat treated with the test sample D (isoacteoside) 2.5 mg/kg, and almost no plaques were found in the brain of the rat treated with the test sample D (isoacteoside) 5mg/kg. Comparing Fig. 12C to Fig. 12E, it is obvious that the plaques in the brain of the rat treated with the test sample D (isoacteoside) were less than that in the brain of the rat treated with Aricept. According to the results, the test sample D (isoacteoside) is effective in inhibiting Aβ forming or cleaning plaques from aggregation.

### Example 11: Neurotransmitters levels in cortex and hippocampus of rats

After the water maze performance tests, all rats were decapitated and their brains were rapidly removed from the skull. According to Glowinski and Iversen, the brain cortex and hippocampus were separated, and then each cortex and hippocampus were weighed and were homogenized by addition of 50 mM Na-phosphate buffer (pH 7.8) for analyzing the concentrations of neurotransmitters and the levels of enzymes.

Table 3 and Table 4 respectively show concentrations of neurotransmitter dopamine (DA) and its metabolites (DOPAC and HVA) in brain cortex and hippocampus of the rats according to Table 2. Referring to Table 3 and Table 4, Aβ1-42 decreases DA levels in the cortex and hippocampus of the rats, and the test sample D (isoacteoside) and Aricept reverse the decrease in DA levels in the cortex and hippocampus of rats caused by Aβ1-42.

**Table 3: DA levels and its metabolites DOPAC and HVA levels in the brain cortex of the rats according to Table 2**

| | DOPAC | HVA | DA |
|---|---|---|---|
| Sham | 16.75±1.70* | 3.71±0.58 | 3.92±0.4** |
| Vehicle | 11.66±1.13 | 2.76±0.48 | 2.33±0.28 |
| D 2.5 mg/kg | 12.53±-3.33 | 2.92±0.58 | 2.67±0.43 |
| D 5 mg/kg | 13.80±3.03 | 2.74±0.69 | 3.84±0.47* |
| Aricept | 10.49±2.38 | 2.42±0.78 | 3.10±0.46 |

**Table 4: DA levels and its metabolites DOPAC and HVA levels in the brain hippocampus of the rats according to Table 2**

| | DOPAC | HVA | DA |
|---|---|---|---|
| Sham | 5.87±0.92 | 3.68±0.41 | 5.36±0:75*** |
| Vehicle | 5.13±0.80 | 3.39±0.36 | 1.00±0.21 |
| D 2.5 mg/kg | 4.98±1.15 | 3.68±0.46 | 5.41±1.30*** |
| D 5 mg/kg | 4.13±0.45 | 3.29±0.25 | 4.07±0.59*** |
| Aricept | 3.19±1.00 | 2.45±0.33 | 1.33±0.37 |

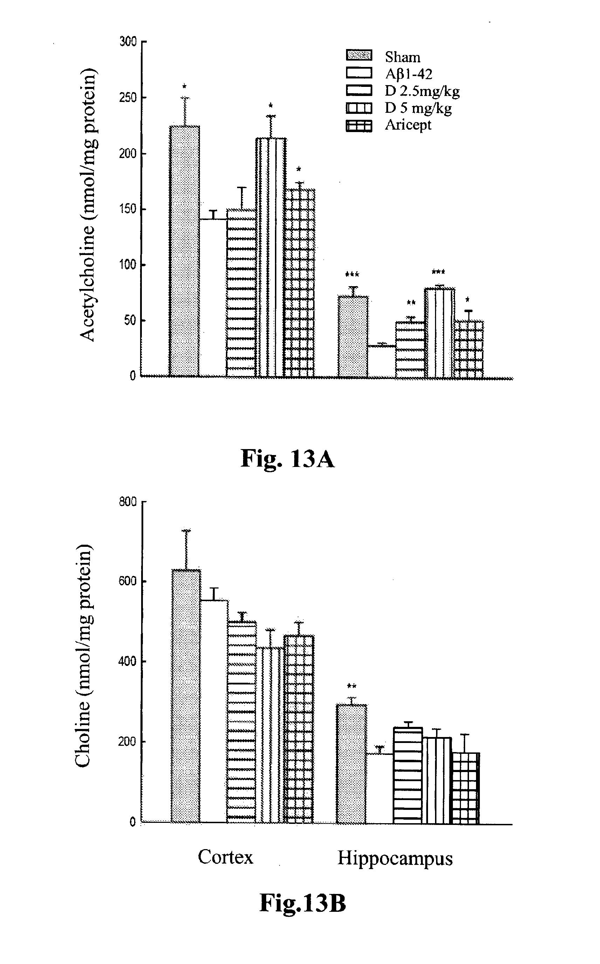

Fig. 13A shows the levels of neurotransmitter acetylcholine in the brain cortex and hippocampus of the rats according to Table 2, and Fig. 13B shows the levels of choline in the brain cortex and hippocampus of the rats according to Table 2. Referring to Fig 13A and Fig. 13B, Aβ1-42 decreases acetylcholine levels in the brain cortex and hippocampus of the rats, and the test sample D (isoacteoside) and Aricept reverse the decrease in acetylcholine levels in the brain cortex and hippocampus of rats caused by Aβ1-42, wherein the test sample D (isoacteoside) 5mg/kg is particularly potent.

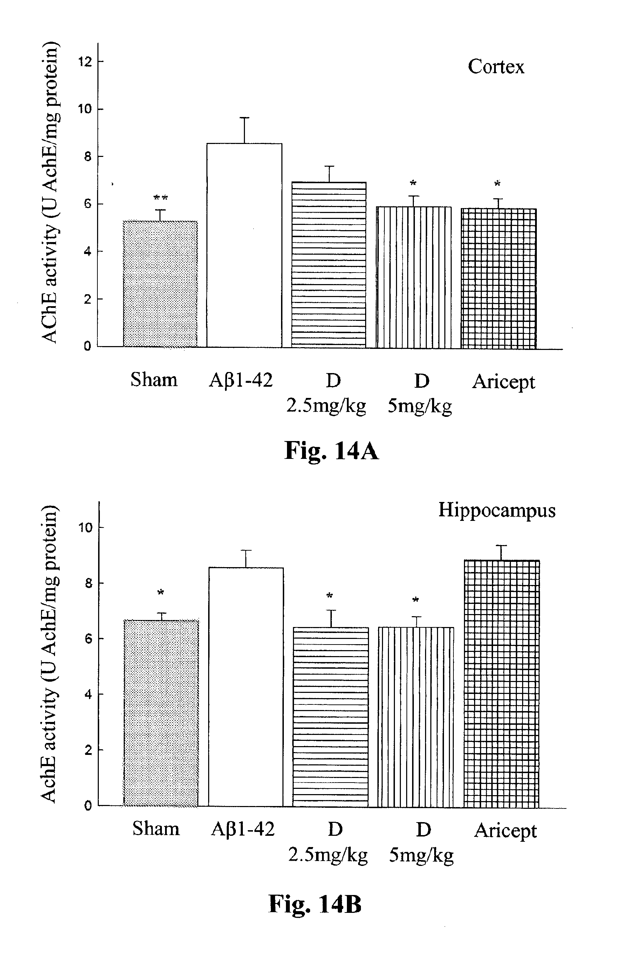

Fig. 14A and Fig. 14B show the activities of acetylcholinesterase in the brain cortex and hippocampus of the rats according to Table 2 respectively. Referring to Fig 14A and Fig. 14B, Aβ1-42 increases acetylcholinesterase activities in the brain cortex and hippocampus of the rats, and the test sample D (isoacteoside) and Aricept reverse the increase in acetylcholinesterase activities in the brain cortex and hippocampus of rats caused by Aβ1-42. Since

Aβ aggregates cause cell damage, and further lead to various diseases or conditions. According to the results from cell studies in Examples 2 to 6, isoacteoside (D) is effective in inhibiting formation, accumulation or aggregation of Aβ, further preventing Aβ from forming aggregates, and thus isoacteoside can be used in preventing or treating Alzheimer's disease or other Aβ-associated diseases or conditions.

The results from animal studies in Examples 7 to 9 show that isoacteoside possesses significant effects on improving memory or learning deficits induced by Aβ. According to Example 10, isoacteoside is effective in inhibiting Aβ forming plaques by aggregation or in cleaning plaques. Besides, according to Example 11, isoacteoside can reverse the decrease in neurotransmitter levels caused by Aβ1-42, and therefore it is effective in improving deficits of memory or learning abilities. The above cell and animal studies results indicate that isoacteoside or its equivalent pharmaceutically acceptable salt can be administered to a person in a daily therapeutically effective amount of 0.2 mg/kg to 4 mg/kg (i.e., a recommended dosage of an adult weighted 60 kg of 12 mg - 240 mg), and used in preventing or treating a disease or condition associated with amyloid β peptides in an individual, wherein said disease or condition is mild cognitive impairment, Lewy body dementia, Down syndrome, Hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, or Pick's disease.

## Claims

1. Isoacteoside or a pharmaceutically acceptable salt thereof for use in preventing or treating a disease or condition associated with amyloid β peptides in an individual, wherein said disease or condition is mild cognitive impairment, Lewy body dementia, Down syndrome, hereditary cerebral hemorrhage with amyloid (HCHWA) Dutch, Parkinsonism-dementia complex on Guam, Cerebral amyloid angiopathy, inclusion body myositis, frontotemporal dementia, age-related macular degeneration, or Pick's disease.

2. The isoacteoside or a pharmaceutically acceptable salt thereof for use according to claim 1, wherein the isoacteoside or a pharmaceutically acceptable salt thereof is used in a dosage equivalent to 0.2 mg - 4.0 mg of isoacteoside or a pharmaceutically acceptable salt thereof per kg of body weight per day.

3. The isoacteoside or a pharmaceutically acceptable salt thereof for use according to claim 1 or 2 as an additive in food, drinks, chewing gums, patches or skin care products.

## Patentansprüche

1. Isoacteosid oder ein pharmazeutisch verträgliches Salz davon zur Verwendung beim Verhüten oder Behandeln einer Krankheit oder eines Zustands bzw. Leidens, die bzw. der bzw. das mit Amyloid-β-Peptiden verbunden ist, in einem Individuum, wobei es sich bei der Krankheit oder dem Zustand bzw. Leiden um eine milde kognitive Beeinträchtigung, Lewy-Körper-Demenz, das Down-Syndrom, hereditäre zerebrale Hämorrhagie mit Amyloidose (hereditary cerebral hemorrhage with amyloid, HCHWA) holländischer Typ, Parkinsonismus-Demenz-Komplex auf Guam, zerebrale Amyloid-Angiopathie, Einschlusskörpermyositis, frontotemporale Demenz, altersbedingte Makuladegeneration oder die Pick-Krankheit handelt.

2. Isoacteosid oder ein pharmazeutisch verträgliches Salz davon zur Verwendung nach Anspruch 1, wobei das Isoacteosid oder ein pharmazeutisch verträgliches Salz davon in einer Dosierung verwendet wird, die 0,2 mg - 4,0 mg von Isoacteosid oder einem pharmazeutisch verträglichen Salz davon pro kg Körpergewicht pro Tag entspricht.

3. Isoacteosid oder ein pharmazeutisch verträgliches Salz davon zur Verwendung nach Anspruch 1 oder 2 als ein Zusatz in Lebensmitteln, Getränken, Kaugummis, Pflastern oder Hautpflegeprodukten.

## Revendications

1. Isoactéoside ou un de ses sels pharmaceutiquement acceptables pour utilisation dans la prévention ou le traitement d'une maladie ou affection associée aux peptides β-amyloïdes chez un individu, ladite maladie ou affection étant un trouble cognitif léger, la démence à corps de Lewy, la trisomie 21, l'hémorragie cérébrale héréditaire avec amylose type néerlandais, le complexe de démence parkinsonienne (syndrome de Guam), l'angiopathie amyloïde cérébrale, la myosite à corps d'inclusion, la démence fronto-temporale, la dégénérescence maculaire liée à l'âge, ou la maladie de Pick.

2. Isoactéoside ou un de ses sels pharmaceutiquement acceptables pour utilisation selon la revendication 1, où l'isoactéoside ou un de ses sels pharmaceutiquement acceptables est utilisé à une posologie équivalente à 0,2 mg-4,0 mg d'isoactéoside ou d'un de ses sels pharmaceutiquement acceptables par kg de poids corporel par jour.

3. Isoactéoside ou un de ses sels pharmaceutiquement acceptables pour utilisation selon la revendication 1 ou 2 en tant qu'additif dans des aliments, des boissons, des gommes à mâcher, des timbres cutanés ou des produits de soin de la peau.
